Europäisches Patentamt

European Patent Office

Office européen des brevets

(11) Numéro de publication: **0 161 956**
**B1**

(12) **FASCICULE DE BREVET EUROPEEN**

(45) Date de publication du fascicule du brevet: **18.01.89**

(51) Int. Cl.⁴: **G 01 S 15/58,** A 61 B 8/06, G 01 P 5/00

(21) Numéro de dépôt: **85400659.0**

(22) Date de dépôt: **02.04.85**

(54) **Procédé de levée d'ambiguité de la mesure par effect Doppler de la vitesse d'un mobile.**

(30) Priorité: **06.04.84 FR 8405485**

(43) Date de publication de la demande:
**21.11.85 Bulletin 85/47**

(45) Mention de la délivrance du brevet:
**18.01.89 Bulletin 89/03**

(84) Etats contractants désignés:
**DE GB NL**

(56) Documents cités:
**EP-A-0 073 418**
**EP-A-0 092 841**

**1973 ULTRASONICS SYMPOSIUM PROCEEDINGS, Monterey, Californie, 5-7 novembre 1973, pages 81-85, IEEE, New York, US; W.C. HAASE et al.: "A directional ratiometric ultrasonic blood flowmeter"**

**ELEKTRONIK, vol. 26, no. 4, 1977, pages 95-100, Munich, DE; A. MARGANITZ: "Mikrowellen-Tachometer misst berührungslos Geschwindigkeitsmessung auch bei Schwebefahrzeugen"**

(73) Titulaire: **CGR ULTRASONIC**
**9, Chaussée de Paris**
**F-77102 Villenoy-les-Meaux (FR)**

(72) Inventeur: **Lannuzel, Olivier**
**THOMSON-CSF SCPI 173, bld Haussmann**
**F-75379 Paris Cedex 08 (FR)**

(74) Mandataire: **Ballot, Paul Denis Jacques et al**
**Cabinet Ballot-Schmit 84, avenue Kléber**
**F-75116 Paris (FR)**

Courier Press, Leamington Spa, England.

# EP 0 161 956 B1

**Description**

L'invention concerne un procédé de levée d'ambiguïté de la mesure par effet Doppler de la vitesse d'un mobile. Le mobile dont il est question dans cette invention doit être appréhendé dans son sens le plus large: en particulier il peut être un fluide s'écoulant dans un tube. Dans le domaine médical cette invention trouve une bonne application, ce fluide est le sang s'écoulant dans une artère ou une veine et le mobile est alors un volume élémentaire de ce sang en un endroit précis du corps humain, par exemple près du coeur. Les problèmes que pose la mesure de la vitesse d'un mobile sont mis en évidence et résolus ici dans une telle application de type médical.

Lorsqu'on mesure la vitesse d'un mobile par effet Doppler en utilisant la technique des ultrasons on émet en direction de ce mobile une impulsion acoustique de durée relativement brève. Cette impulsion se propage depuis une sonde émettrice à travers le milieu qui entoure le mobile et se réfléchit sur ce mobile en une impulsion acoustique. Elle se rétrodiffuse dans toutes les directions et en particulier en direction d'une sonde de mesure. Les sondes utilisées sont généralement d'un type réversible ce qui fait que c'est la même sonde qui émet l'impulsion acoustique et qui reçoit ultérieurement l'impulsion réfléchie. Si l'impulsion émise vibre à une fréquence fo l'impulsion réfléchie vibre à une fréquence décalée de fo par effet Doppler. L'amplitude de ce décalage mesure la composante radiale de la vitesse du mobile selon l'axe aller-retour de propagation de ces impulsions. Comme il n'y a pas que le mobile qui réfléchit l'impulsion il est connu d'ouvrir une fenêtre temporelle de réception au bout d'une durée déterminée après le début de l'émission de l'impulsion, pour recevoir la vibration acoustique réfléchie qui correspond au mobile et à lui seul. Cette durée déterminée correspond au rapport de la distance séparant le mobile et la sonde à la vitesse de propagation des impulsions dans le milieu qui entoure le mobile.

En fait, au lieu d'émettre une impulsion unique et de recevoir une impulsion unique on émet périodiquement des impulsions acoustiques et on reçoit périodiquement les impulsions réfléchies. Cette périodicité d'émission est liée d'une part à la volonté de l'utilisateur de connaître la vitesse du sang en différents endroits d'un organe étudié, dans ce cas on déplace d'une mesure à l'autre la position dans le temps de la fenêtre de réception. D'autre part, toutes les particules d'un volume élémentaire d'un fluide ne se déplaçant pas à la même vitesse il convient de calculer la vitesse moyenne du volume élémentaire à un endroit considéré et donc d'effectuer l'analyse spectrale de l'impulsion réfléchie ce qui dans l'état actuel de la technique ne peut pas se faire avec la réception d'une seule impulsion mais ne peut se faire qu'en combinant la mesure de plusieurs impulsions réfléchies successives. La mise en oeuvre d'un tel procédé est par exemple présentée dans la demande de brevet européen EP—A—0092841.

La conséquence immédiate de cette périodicité est de limiter le décalage Doppler admissible qui soit directement mesurable. En effet, tout se passe comme si l'on soumettait le phénomène étudié, le sang en mouvement, à une illumination stroboscopique. Les volumes élémentaires dont la vitesse provoque un décalage ou glissement Doppler sensiblement égal à la fréquence de récurrence sont sensiblement vus comme ne se déplaçant pas. Les volumes élémentaires se déplaçant légèrement plus vite qu'une vitesse nominale, pour laquelle le glissement Doppler est égal à la demi-fréquence de récurrence, sont même interprêtés comme se déplaçant en sens inverse de leur déplacement réel. Le phénomène se reproduit bien entendu pour tout glissement Doppler qui est un multiple entier de la demi-fréquence de récurrence. Or, l'utilisateur n'a pas accès à la vitesse vraie il n'a accès qu'à la vitesse mesurée: ceci signifie que pour cet utilisateur il y a toujours une ambiguïté. Cette ambiguïté pose la question de savoir quelle est la vitesse vraie du volume élémentaire étudié connaissant la vleur mesurée obtenue.

Il est connu dans l'état de la technique deux types de solutions qui tentent de répondre à ce problème. La première solution consiste à augmenter la fréquence de récurrence $F_r$ de manière à porter au plus haut possible le seuil d'ambiguïté. Si l'on appelle $f_m$ la mesure du glissement Doppler correspondant à une vitesse donnée, la vitesse nominale, c'est-à-dire celle admissiblement mesurable, est telle que $f_m=F_r/2$. En conséquence, plus la fréquence de récurrence augmente plus le seuil d'ambiguïté est repoussé vers les valeurs élevées: si possible au-delà de la gamme des vitesses que l'on s'attend à mesurer. Cette première technique présente deux inconvénients. Le premier inconvénient est d'ordre physiologique car plus la fréquence de récurrence augmente plus le nombre des impulsions envoyées dans le corps du patient est élevé. Il n'est pas certain, qu'à hautes doses, des excitations ultrasonores ne soient pas destructrices de certaines cellules du corps humain. Deuxièmement le fait d'augmenter la fréquence de récurrence diminue corrélativement la période qui sépare deux impulsions d'excitation successives. Ceci limite la plage de réglage dans le temps de la position de la fenêtre temporelle de réception. En définitive, c'est la profondeur d'exploration depuis la sonde qui est réduite.

Dans certains cas il peut même y avoir des erreurs de mesure liées au fait que pendant la fenêtre temporelle de réception on reçoit un signal qui correspond à la réception d'une impulsion émise et réfléchie à une profondeur donnée du milieu étudié et qui correspond également à la réception d'une impulsion précédente mais réfléchie dans une zone plus profonde de ce milieu. Autrement dit, si l'obstacle de profondeur d'exploration peut être contourné, il reste que le résultat de la mesure rend compte en même temps de l'interférence de parties plus profondes ce qui le fausse. Dans un exemple pratique, si la fréquence fo vaut 7 MHz et si la composante radiale de la vitesse du sang est de l'ordre de 3 m/s, dans le corps humain où la vitesse de propagation est de l'ordre de 1500 m/s, le décalage Doppler correspondant est de l'ordre de 30 KHz et la profondeur maximum d'exploration est de l'ordre de 2,5 cm. Or, une vitesse radiale de 3 m/s est représentative d'une vitesse d'écoulement du sang de l'ordre de 5 m/s si l'axe de propagation des impulsions est orienté sensiblement à 60° par rapport à la direction moyenne du sang à l'endroit de la mesure (ce qui correspond à un mode général d'expérimentation). Comme les patients à examiner sont plutôt des malades atteints d'affections cardiaques, la vitesse réelle du sang peut être notablement plus forte (du fait notamment de sténose aortique). Donc même dans ce cas où la fréquence de récurrence est importante et où la profondeur d'exploration est réduite des ambiguïtés de mesures sont quand même rencontrées.

Une deuxième solution technique consiste à faire baisser la fréquence du signal acoustique émis. Si fo est cette fréquence acoustique le glissement Doppler $f_d$ peut s'exprimer sous la forme:

$$f_d = 2 \cdot v \cdot fo/c$$

où $v$ est la vitesse à mesurer et où $c$ est la vitesse de propagation de l'impulsion acoustique dans le milieu. Pour une vitesse $v$ donnée, $f_d$ sera d'autant plus faible que $f_0$ est lui-même faible. L'inconvénient de ce choix est qu'il y a une perte importante de sensibilité sur la détermination de $f_d$.

L'invention a pour objet de remédier aux inconvénients cités en proposant un procédé où l'on module par un signal à une fréquence connue le signal représentatif du glissement Doppler à mesurer. Ceci a pour effet de transposer dans le domaine des fréquences les seuils d'ambiguïté et permet d'élaborer une mesure dite décalée de la valeur vraie. On retrouve la valeur vraie en corrigeant la valeur décalée d'une grandeur correspondant à la fréquence de modulation introduite.

L'invention a pour objet un procédé de levée d'ambiguïté de la mesure de la vitesse V d'un mobile (1) par effet Doppler, qui comprend les étapes suivantes:

l'émission (6, 7, 8) en direction du moblie d'un signal acoustique de fréquence fo, modulé en impulsions, la fréquence de récurrence des impulsions étant Fr,

la réception (50, 9) du signal acoustique S(t) rétrodiffusé par le mobile,

la démodulation complexe (9) du signal S(t) par un signal à la fréquence d'émission fo pour obtenir un signal démodulé Z(t),

l'extraction, par analyse spectrale (10) du signal démodulé Z(t), de la composante à la fréquence Doppler représentative de la vitesse du mobile,

ledit procédé étant caractérisé en ce qu'il comprend, pour éliminer l'ambiguïté de mesure due à l'échantillonage du signal démodulé Z(t) à la fréquence de récurrence Fr,

a) la comparaison de la fréquence Doppler issue des moyens d'analyse spectrale (10) à une valeur de seuil donnée liée à l'apparition de l'ambiguïté, et en cas de dépassement du seuil, les étapes suivantes effectuées selon un processus récursif;

b) la modulation (11) du signal démodulé Z(t) par un signal oscillant à une fréquence de détermination FD, de manière à obtenir une fréquence Doppler transposée fa d'une grandeur connue dans une bande de fréquence inférieure à la moitié de la fréquence de récurrence Fr;

c) l'extraction, par analyse spectrale (10), de la fréquence Doppler transposée fa et la correction de ladite fréquence Doppler transposée par une grandeur fc correspondant à la fréquence de détermination pour calculer la fréquence Doppler varie fv;

d) la comparaison de la fréquence Doppler transposée à la valeur de seuil donnée et la poursuite du processus à partir de l'étape (b) précédente en cas de dépassement du seuil, et ceci jusqu'à l'obtention d'une fréquence Doppler vraie fv non ambigüe.

L'invention sera mieux comprise à la lecture de la description qui suit et à l'examen des figures qui l'accompagnent. Cette description n'est donnée qu'à titre indicatif et nullement limitatif de l'invention. Sur les figures les mêmes repères désignent les mêmes éléments. Elles représentent:

figure 1, une représentation schématique d'un dispositif mettant en oeuvre le procédé de l'invention,

la figure 2, le diagramme du spectre de fréquence d'un signal à analyser par le procédé de l'invention,

figure 3a et 3b, des diagrammes temporels de l'historique des vitesses d'un mobile et des signaux mesurés correspondants intervenant dans le procédé de l'invention;

figure 4, un schéma d'un circuit d'analyse spectrale particulièrement mis en oeuvre dans l'invention;

figure 5, un diagramme temporel de la vitesse décalée et de la vitesse vraie;

figure 6, un organigramme montrant l'articulation de différentes phases du procédé de l'invention;

figure 7, une représentation symbolique de la mesure de glissement Doppler;

figure 8, un organigramme permettant l'initialisation du procédé de l'invention.

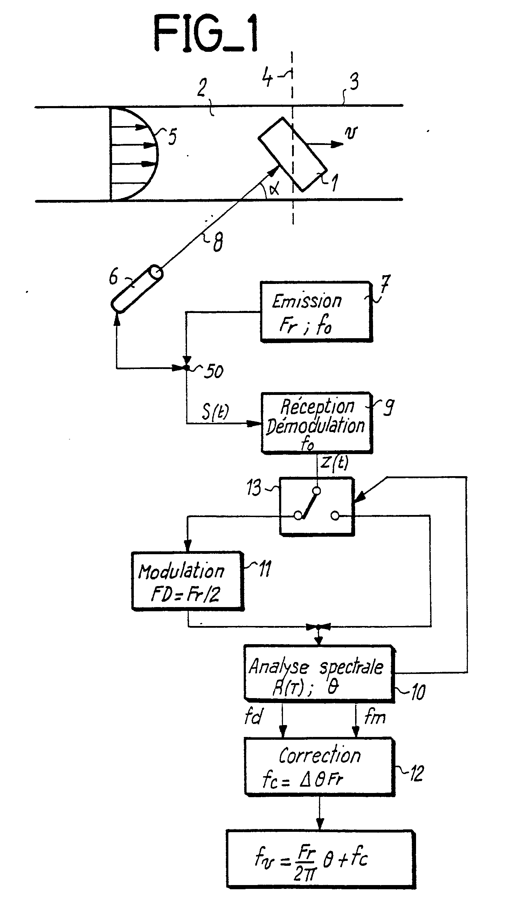

La figure 1 représente le schéma d'un dispositif mettant en oeuvre le procédé de l'invention. Il permet de mesurer la vitesse moyenne d'un volume élémentaire 1 d'un fluide 2 circulant dans un tube 3. Le volume élémentaire 1, qui est un mobile au sens le plus large, est un ensemble de particules du fluide 2 se trouvant en un endroit particulier du tube 3: la section 4. Le fluide 2 ayant un diagramme de vitesse symboliquement représenté en 5 se déplace en moyenne à une certaine vitesse $v$ que l'on cherche à mesurer. Pour ce faire on émet au moyen d'une sonde 6 d'un type piézoélectrique et d'un générateur 7 une série d'impulsions acoustiques. Ces impulsions acoustiques vibrent à une fréquence acoustique fo et sont émises périodiquement à une fréquence de récurrence $F_r$. L'axe 8 de propagation aller-retour des ondes acoustiques entre le volume 1 et la sonde 6 fait un angle $\alpha$ avec la direction moyenne d'écoulement du fluide 2. Chaque impulsion qui insonifie le volume 1 est rétrodiffusée en direction de la sonde 6 en vibrant

3

EP 0 161 956 B1

autour de la fréquence fo, et en étant décalé de cette fréquence d'une grandeur proportionnelle à la vitesse d'écoulement et au cosinus de l'angle α. Ceci étant posé l'influence de l'angle α sera négligée dans la suite de la description.

La sonde 6 est d'un type réversible: c'est-à-dire que le cristal piézoélectrique qu'elle comporte délivre une impulsion acoustique quand il est soumis à un signal électrique au moment de l'émission, et délivre un signal électrique quand il est soumis à une excitation acoustique au moment de la réception. Hors les temps d'émission le signal acoustique reçu est transmis vers un récepteur 9 par tout moyen, en particulier au travers d'un duplexeur 50. Dans le récepteur 9 le signal reçu est filtré temporellement et est démodulé par un signal à la fréquence fo. Le résultat de cette démodulation est de faire apparaître un signal représentatif des glissements Doppler seuls à l'endroit de la section 4.

Dans les procédés connus de l'état de la technique on effectue l'analyse spectrale de ce glissement Doppler dans un dispositif analyseur de spectre 10. Quand ce glissement Doppler est supérieur à la moitié de la fréquence d'échantillonnage il se produit un repliement de fréquence de ce signal de glissement Doppler qui est mal interprété par le circuit 10. Ce qui caractérise l'invention c'est que le signal introduit dans le circuit d'analyse 10 n'est pas le signal démodulé par le récepteur 9 mais plutôt ce signal démodulé ayant subi une modulation dans un modulateur 11 par un signal à une fréquence dite de détermination FD. Cette modulation revient à transposer vers les basses fréquences, d'une grandeur FD, le spectre du signal de glissement Doppler. Avec un filtrage approprié on récupère ce spectre transposé seul. La fréquence FD étant connue il est alors possible d'affecter le résultat $f_d$ délivré par l'analyseur 10 d'une correction $f_c$ connue dans un circuit de correction 12.

Comme il y a une correspondance biunivoque entre la vitesse $v$ du mobile 1 et la valeur du glissement Doppler vrai correspondant, on n'évoquera plus dans la suite de cet exposé que les fréquences de ces glissements Doppler. Ainsi, la fréquence vraie $f_v$ est la somme de la fréquence dite décalée $f_d$ délivrée par le circuit d'analyse 10 et de la fréquence de correction $f_c$ délivrée par le circuit 12. La fréquence mesurée $f_m$ correspond à celle délivrée par l'analyseur 10 lorsque l'on ne fait pas intervenir le modulateur 11. On verra dans la suite de cet exposé que l'invention permet d'interposer ou non le modulateur 11 selon que le glissement Doppler à mesurer se situe dans une gamme avec ou sans ambiguïté. Symboliquement, un commutateur 13 dont la position dépend de la valeur mesurée par l'analyseur 10 permet d'interposer ou de shunter ce modulateur 11 entre le récepteur 9 et l'analyseur 10.

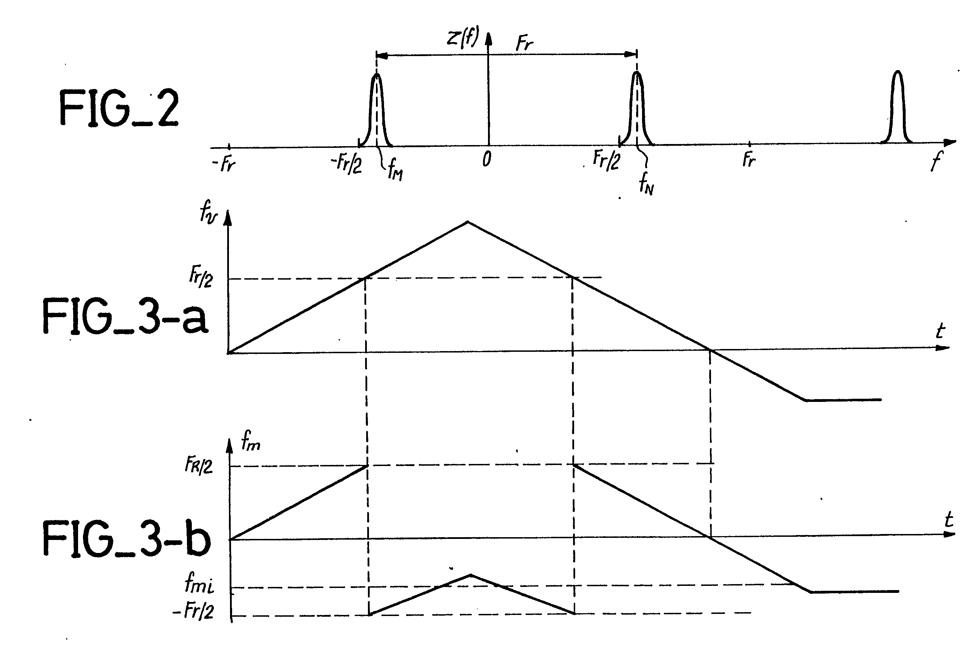

La figure 2 représente le diagramme Z(f) du spectre Doppler du signal Z(t) délivré après démodulation dans le récepteur 9. L'insonification périodique du mobile 1 revient à échantillonner Z(t) puisque l'on prélève Z(t) à chaque période. Du fait de cet échantillonnage à la fréquence $F_r$, le spectre de Z(t) est périodique de périodicité $F_r$. La figure 2 représente un spectre de Z(f) qui n'est pas monofréquence. Il s'étend quelque peu de part et d'autre d'une fréquence moyenne $f_M$ et indique ainsi que toutes les parties du mobile 1 ne se déplacent pas à la même vitesse, mais se déplacent chacune à des vitesse proches de la vitesse moyenne. Le diagramme de la figure 2 indique encore que c'est la fréquence $f_M$ qui va être analysée dans l'analyseur 10, sans que l'on puisse savoir si $f_M$ est la fréquence vraie ou une fréquence ambiguë $f_N$, décalée de $f_M$ d'un nombre entier de fois la fréquence d'échantillonnage.

Sur les figures 3a et 3b sont représentées respectivement et en correspondance dans le temps les évolutions de la fréquence vraie $f_v$ et de la fréquence mesurée $f_m$. Dans un exemple, à une fréquence $f_{mi}$ la mesure correspond à une vitesse négative alors qu'en fait la vitesse est tout simplement supérieure à la vitesse nominale mesurable par l'analyseur 10. Par ailleurs l'ambiguïté de signification de la mesure $f_{mi}$ est mise en évidence puisque cette fréquence mesurée correspond à deux valeurs possibles différentes à la vitesse d'écoulement.

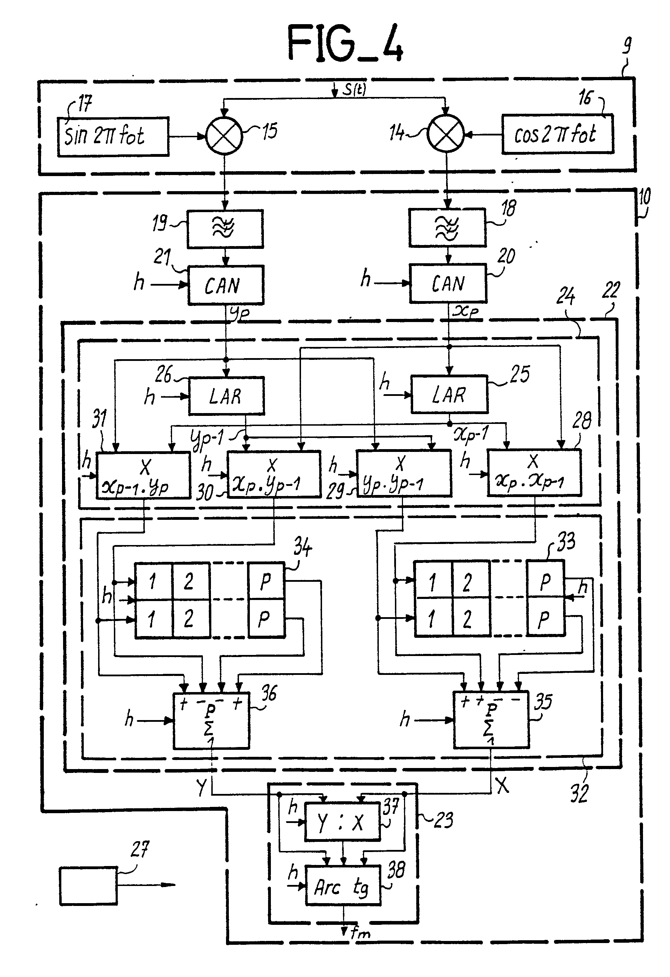

La figure 4 présente un dispositif d'analyse spectrale mettant en oeuvre un procédé pour calculer la vitesse moyenne d'un volume élémentaire 1 d'un fluide. Ce procédé a pour base que la vitesse moyenne d'écoulement d'un volume élémentaire d'un fluide correspond au moment d'ordre un de la transformée de Fourier du glissement Doppler provoqué par ce volume élémentaire, et le fait que ce moment d'ordre un de la transformée de Fourier est égal à la dérivée d'ordre un de la fonction d'autocorrélation, R(t), du signal temporel de glissement Doppler. Hormis les remises en forme et remises à l'échelle auxquelles on soumet le signal S(t) transmis par la sonde 6 au récepteur 9, ce récepteur comporte deux démodulateurs 14 et 15 recevant chacun le signal S(t) sur leur première entrée. Sur leur deuxième entrée ils reçoivent respectivement des signaux en quadrature, cos 2πfo t et sin 2πfo t délivrés par deux oscillateurs en quadrature 16 et 17. Ces deux démodulateurs effectuent une transposition du spectre Doppler d'une grandeur égale à $f_0$.

Ils effectuent une démodulation dite en quadrature car les signaux qui les attaquent sur leur deuxième entrée sont en quadrature de phase l'un par rapport à l'autre.

Dans l'analyseur 10 on fait passer chacun des signaux démodulés à travers un filtre passe-bas respectivement 18 et 19, et on les quantifie dans des échantillonneurs respectivement 20 et 21, comprenant chacun un échantillonneur-bloqueur suivi d'un convertisseur analogique-numérique (CAN). On obtient alors un signal complexe échantillonné, dont la partie réelle $x_p$ est délivrée par l'échantillonneur 20, et dont la partie imaginaire $y_p$ est délivrée par l'échantillonneur 21. Ainsi à chaque échantillon récurrent du signal Z(t) on a prélevé un échantillon quantifié $z_p = x_p + jy_p$. Le circuit d'analyse spectrale 10 comporte encore un circuit 22 pour calculer la fonction d'autocorrélation R(T) du signal Z(t) et un circuit 23 pour calculer la dérivée d'ordre un de cette fonction d'autocorrélation. Comme le signal Z(t) est échantillonné et complexe,

4

la fonction d'autocorrélation devient discrète, r(T), et complexe. La partie réelle de r(T) est X, sa partie imaginaire étant Y.

Le résultat élaboré dans le procédé évoqué permet d'écrire que la fréquence moyenne $f_m$ mesurée par un tel dispositif d'analyse spectrale est donnée par la formule suivante:

$$f_m = \frac{F_r}{2\pi} \text{Arg}(r(T)) \text{ avec } T = \frac{1}{F_r}.$$

Il convient donc de calculer l'argument de cette fonction autocorrélation discrète $r(1/F_r)$. Or, $r(1/F_r)$ peut s'écrire:

$$r(\frac{1}{F_r}) = Z = X + jY = \overset{P}{\Sigma}((x_p + jy_p)*(x_{p-1} + jy_{p-1})) = \overset{P}{\Sigma}(z_p* . z_{p-1})$$

Dans cette expression $p$ est l'indice temporel d'un échantillon du signal démodulé délivré par les convertisseurs analogique'numérique 20 et 21, $p-1$ étant le rang de l'échantillon qui iest apparu juste avant l'échantillon de rang $p$. Le nombre P correspond à un optimum de précision de la mesure. Dans un exemple P vaut environ une centaine. L'exposant * indique la conjugaison complexe.

Autrement dit la connaissance d'un certain nombre d'échantillons $z_p$ permet de calculer la fonction d'autocorrélation du signal S(t) démodulé ainsi que sa dérivée d'ordre un. Ceci est exprimé par les grands Sigma qui se trouvent à la fin de l'expression ci-dessus. Or l'argument $\theta$ d'un nombre complexe Z (représentatif de la fonction d'autocorrélation) est égal, sous certaines restrictions, à l'arc-tangente du rapport de sa partie imaginaire à sa partie réelle. Ceci revient à écrire:

$$\theta = \text{Arg}(Z) = \text{Arctg}\frac{Y}{X}$$

Cette égalité n'est vraie que dans le domaine de définition sans discontinuité de l'arc-tangente, c'est-à-dire entre $-\pi/2$ et $+\pi/2$. Si l'argument de Z est compris entre $-\pi$ et $-\pi/2$, il convient de retrancher la valeur $\pi$ au calcul effectué au moyen de l'arc-tangente; si l'argument de Z est compris entre $+\pi/2$ et $+\pi$ il convient d'ajouter $\pi$. On lève ainsi l'indétermination sur $\theta$. Ceci étant dit on va expliquer dans un premier temps comment l'analyseur 10 calcule en temps réel la fonction d'autocorrélation Z ainsi que son argument $\theta = \text{Arg}(Z)$. Dans un deuxième temps on montrera quelle est l'incidence, sur ce calcul, de la modulation de S(t) par une fréquence de détermination FD bien précise qui est égale à la moitié de la fréquence de récurrence. On expliquera enfin dans le cadre de ce calcul quel grand intérêt procure ce choix particulier.

En revenant à la formule permettant le calcul de la fonction d'autocorrélation en fonction des valeurs des échantillons complexes $z_p = x_p + jy_p$ on peut écrire:

$$X = \overset{P}{\Sigma}(x_p . x_{p-1} + y_p . y_{p-1}) \text{ et } Y = \overset{P}{\Sigma}(y_p . x_{p-1} - y_{p-1} . x_p)$$

Le circuit 22 comporte des moyens de retard 24 pour connaître, à chaque impulsion reçue, les grandeurs $x_p$ et $y_p$ du signal complexe échantillonné correspondant à l'échantillon de rang $p$ et pour connaître les mêmes éléments de l'échantillon immédiatement précédent c'est-à-dire de rang p−1. Les moyens de retard 24 comportent deux lignes à retard respectivement 25 et 26 reliées en cascade aux sorties des échantillonneurs 20 et 21. Ces lignes à retard 25 et 26 ne délivrent à leurs sorties l'information qu'elles reçoivent à un instant $t$ qu'au bout d'un temps t+T, où T correspond à la période de récurrence précitée. Dans la pratique ces lignes à retard peuvent être constituées de registres à décalage recevant sur leurs entrées en parallèle les informations binaires du signal quantifié délivré par les échantillonneurs, et restituant ces informations sur leurs sorties en parallèle sous l'effet d'une impulsion d'horloge $h$ élaborée par un séquenceur 27. Ce séquenceur 27 est synchronisé avec l'émission des impulsions acoustiques. La fréquence de l'échantillonnage est alors égale à la fréquence de récurrence puisque les convertisseurs 20 et 21 sont également piloté par le signal $h$.

Les moyens de retard 24 comportent en outre un jeu de multiplicateurs 28 à 31 recevant sur leurs entrées les parties réelles, x, ou imaginaires, y, du signal complexe échantillonné, correspondant à un échantillon donné et à un échantillon précédent, pour effectuer, au rythme imposé par le séquenceur 13, respectivement les multiplications suivantes:

$$x_p . x_{p-1}, \quad y_p . y_{p-1}, \quad x_p . y_{p-1}, \quad x_{p-1} . y_p.$$

Le circuit 22 comporte, en cascade avec les moyens de retard 24, des moyens sommateurs-accumulateurs 32 comportant un jeu de deux registres à décalage 33 et 34 reliés respectivement à deux sommateurs-accumulateurs 35 et 36. Ces registres à décalage contiennent chacun 2P . cases (P correspond

# EP 0 161 956 B1

au nombre optimum d'échantillons). Ces registres reçoivent les produits élaborés par les multiplicateurs 28 et 29 d'une part et 30 et 31 d'autre part. A chaque impulsion du séquenceur 27, un couple de produits réels $(x_p \cdot x_{p-1}$ et $y_p \cdot y_{p-1})$ et un couple de produits imaginaires $(x_p \cdot y_{p-1}$ et $x_{p-1} \cdot y_p)$ sont introduits respectivement dans ces registres à décalage, tandis qu'un couple de produits réels ou de produits imaginaires correspondants mais de rang antérieur

$$\text{(de rang } p-P;\ x_{p-P} \cdot x_{p-1-P},\ y_{p-P} \cdot y_{p-1-P};\ \text{et } x_{p-P} \cdot y_{p-1-P},\ x_{p-1-P} \cdot y_{p-P})$$

sont extraits de ces registres. Les sommateurs-accumulateurs 35 et 36 additionnent alors les produits introduits en même temps qu'ils soustraient les produits extraits. Une fois que l'on a acquis les P premiers échantillons, premièrement chacun des registres à décalage reçoit en introduction un produit correspondant au rang de l'échantillon suivant et deuxièmement délivre en extraction les produits correspondants à des rangs antérieurs de P. Dans les sommateurs-accumulateurs 35 et 36 les produits introduits et extraits sont admis sur des entrées affectées d'un signe particulier selon qu'il convient d'additionner ou de soustraire ces produits.

Dans le cas du sommateur-additionneur 35, qui délivre la partie réelle X de la fonction d'autocorrélation, tous les nouveaux produits introduits dans les registres sont affectés du signe+tandis que tous les anciens produits extraits sont affectés d'un signe −. Il en résulte que le sommateur-accumulateur 35 contient en permanence, donc en temps réel, la partie réelle de la fonction d'autocorrélation qui aura porté sur les P échantillons qui précèdent l'instant auquel on prélève le contenu de ce sommateur-accumulateur 35. Un raisonnement similaire peut être fait pour le sommateur-accumulateur 36 en remarquant cependant qu'il est spécialisé dans le calcul de la partie imaginaire de la fonction d'autocorrélation dans lequel le Sigma comporte une soustraction des produits. Tous les circuits des moyens 32 sont pilotés par le séquenceur 27. Compte tenu d'une fréquence d'échantillonnage de l'ordre de la dizaine de KHz le temps de calcul pour ces circuits est de l'ordre de 100 microsecondes. Dans l'état actuel de la technologie les rapidités de calcul des circuits intégrés sont telles que ces opérations ne présentent aucune difficulté. Dans la pratique ces circuits peuvent être remplacés par un microprocesseur dont l'algorithme général reproduit celui qui vient d'être décrit.

Ce microprocesseur en particulier peut effectuer de plus l'opération représentée par le circuit 23 de calcul de la dérivée d'ordre un de la fonction d'autocorrélation. Dans ce circuit 23 un diviseur 37 effectue le rapport de la partie imaginaire Y à la partie réelle X. Une table d'arc-tangente 38 reliée au diviseur 27 délivre l'argument de la fonction d'autocorrélation en fonction du rapport élaboré par le diviseur 37. Pour lever l'indétermination liée à un calcul par l'arc-tangente; la table 38 reçoit également la valeur X et la valeur Y. En effet, si l'argument de Z est compris entre $-\pi$ et $-\pi/2$ ceci signifie que X et Y sont négatifs. Si l'argument est compris entre $+\pi/2$ et $\pi$ ceci signifie que X est négatif et que Y est positif. En conséquence, la table 38 reçoi en adresse d'une part la valeur du rapport Y/X et d'autre part des informations concernant le signe de X et de Y. Elle peut effectuer sans indétermination le calcul de l'argument recherché et plus généralement le calcul de la grandeur $f_m$.

Pour un signal S(t) donné le résultat de sa modulation par un signal à une fréquence de détermination FD peut s'exprimer sous la forme suivante:

$$(S\ (t)\ \text{modulé}) = S(t) \cdot e^{-j2\pi FDt}$$

Dans le cas qui nous préoccupe, étant donné que le signal S(t) est échantillonné à des dates $t = p/F_r$, où $p/F_r = p \cdot T$, cela correspond à un échantillonnage périodique de période T. L'expression de S(t) modulé et échantillonné devient:

$$(S(t)\ \text{modulé échantillonné}) = S(t) \cdot e^{-j2\pi FD/F_r \cdot p}.$$

ou encore en exprimant S(t) sous sa forme quantifiée:

$$(S(pT)\ \text{modulé échantillonné}) = z_p e^{-j2\pi FD/F_r \cdot p}\ \text{où}\ z_p = x_p + j y_p.$$

A ce stade de la description on découvre un grand intérêt qu'il y a à faire $FD = F_r/2$. En effet dans ce cas l'exponentielle complexe représentative de la modulation devient:

$$e^{-j2\pi F_r/2F_r P} = e^{-j\pi p} = (-1)^p$$

Ce résultat particulièrement simple permet d'exprimer que si le signal S(t) est modulé, l'échantillon quantifié $z_p$ est remplacé par $(-1)^p \cdot z_p$. Autrement dit dans le calcul de la fonction d'autocorrélation donnée plus haut:

$$r\!\left(\frac{1}{F_r}\right) = \Sigma z^*_p \cdot z_{p-1}$$

6

chaque échantillon $z_p$ doit être multiplié par $+1$ ou $-1$ selon que le rang de $p$ est pair ou impaire. En fait on peut même se passer de cette opération qui consisterait à changer le signe, une fois sur deux au rythme de leur arrivée, des valeurs $x_p$ et $y_p$ délivrées par les convertisseurs analogiques-numériques 20 et 21. En effet, il convient de remarquer que:

$$(-1)^p \cdot (-1)^{p-1} = -1$$

Ce qui signifie tout simplement que la modulation de S(t) par un signal à fréquence de détermination égale à $F_r/2$ revient à changer le signe de la fonction d'autocorrélation par rapport à la valeur qu'elle aurait eu si on n'avait pas modulé. Autrement dit Z modulé égale $-Z$. L'intérêt de cette constatation consiste à remarquer qu'en définitive l'introduction de la modulation pourra se faire à posteriori, c'est-à-dire en connaissance de la valeur de Z (ou de son argument $\theta$ ce qui est équivalent). Le décalage affectant $\theta$ du fait de la modulation doit être compensé pour retrouver la valeur vraie. Dans le cas présent où FD=$F_r/2$, cette correction vaut$\pm\pi$. La question se pose alors de savoir, puisque l'on peut décider après coup de "moduler" ou non, à partir de quelle valeur il convient de le faire.

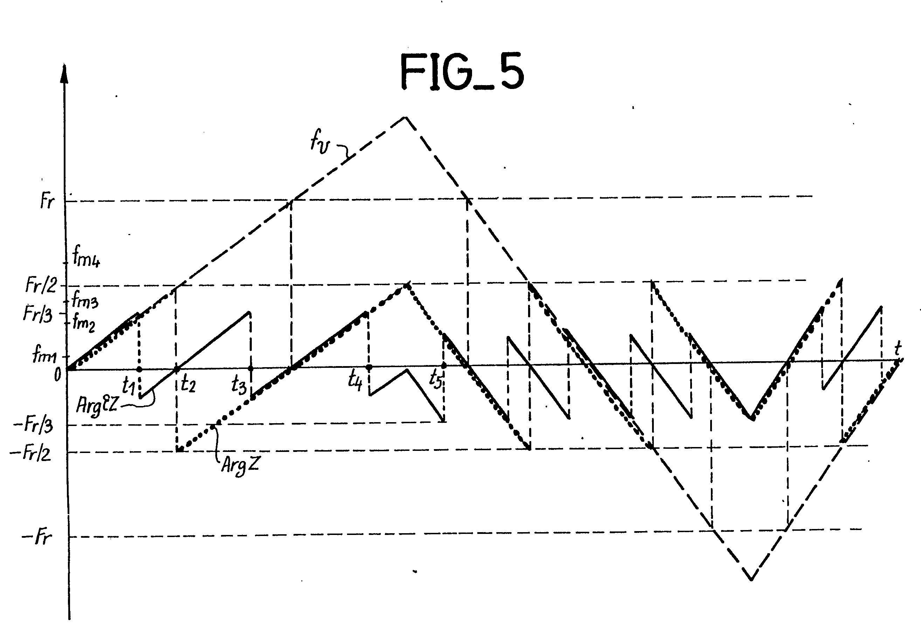

Sur la figure 5 on a représenté un phénomène qui correspond à un mobile, ou volume élémentaire d'un fluide, dont la valeur de la vitesse évolue continuement dans le temps. Ceci pourrait représenter la vitesse d'écoulement du sang dans le coeur d'un patient. Ce qui va être dit pour ce phénomène est bien entendu applicable au cas du coeur même si la vitesse du sang y est en général orientée toujours dans un seul sens.

Le diagramme de la figure 5 présente trois courbes: la première dessinée en tirets représente la vitesse vraie $f_v$ (tout est exprimé ici sous la forme de glissement Doppler moyen c'est-à-dire en termes fréquentiels). Elle représente en définitive le signal que l'on cherche à produire. Un deuxième courbe dessinée en pointillés est proportionnelle à la variation de l'argument de Z: c'est le résultat $f_m$ que délivre le circuit 22 lorsqu'il n'y a pas de modulation. Cette deuxième courbe présente les ambiguïtés que l'on a rencontrées dans la figure 3b. La troisième courbe dessinée en trait plein est proportionnelle à Arg($\varepsilon$Z) avec $\varepsilon = \pm 1$ selon que l'on a, ou que l'on a pas modulé. Quand la courbe en pointillés est colinéaire à la courbe en trait plein on n'a pas modulé, quand elle n'est pas colinéaire à celle-ci on a modulé.

Le traitement auquel est soumis Arg($\varepsilon$Z) est un traitement effectué par un microprocesseur. La conduite de ce traitement y est plus souple que dans un circuit câblé. On peut cependant construire un tel circuit câblé à partir de la description de l'algorithme de traitement décrit ci-dessous. On retient la convention suivante portant sur le signe de $\varepsilon$:

$$\text{si } \varepsilon = +1, \quad \theta = \text{Arg}(Z) = \text{Arctg}\frac{Y}{X}$$

$$\text{si } \varepsilon = -1, \quad \theta = \text{Arg}(-Z) = \text{Arctg}\frac{Y}{X} - \pi \text{ si Y est positif}$$

ou

$$\theta = \text{Arg}(-Z) = \text{Arctg}\frac{Y}{X} + \pi \text{ si Y est négatif}$$

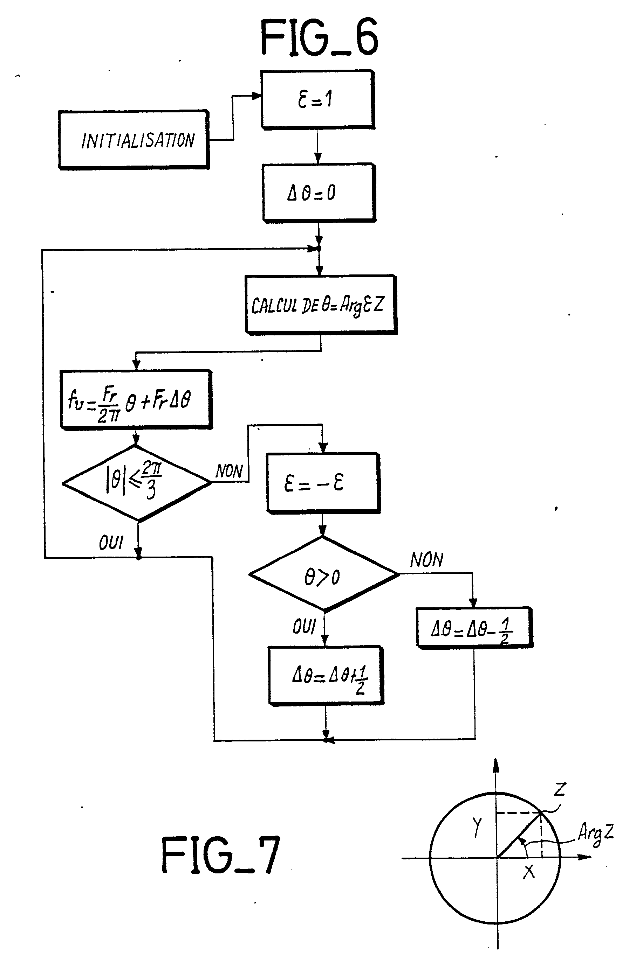

Cette convention nous permet de passer de la courbe en pointillés à la courbe en traits pleins. En fait, la distinction portant sur le signe de $\pi$ est équivalente à tester le signe de Y. La convention sera plus facilement comprise à l'examen conjoint des figures 5 et 7. Cette dernière représente le calcul théorique d'un argument à partir de la valeur de l'arc-tangente. Ainsi, pour une première valeur de glissement Doppler $f_{m1}$ comprise entre zéro et $F_r/4$, X et Y sont positifs et $\theta$ égale Arctg Y/X; $\varepsilon$ égale 1, on ne module pas.

Pour une deuxième valeur mesurée $f_{m2}$ comprise entre FR/4 et une certaine valeur de seuil, égale ici à $F_{r/3}$, Z se retrouve dans le cadran supérieur gauche de la figure 7. Dans ce cas $\theta$ est redonné dans les mêmes conditions: on ne module pas. Par contre pour une troisième valeur mesurée $f_{m3}$, supérieure au seuil $F_r/3$ mais inférieure à $F_r/2$ on a décidé de moduler, donc on a changé le signe de $\varepsilon$ alors que Y était positif. On remarque en effet que Y est positif jusqu'à ce que $f_{m3}$ atteigne $F_r/2$. On vérifie ainsi la convention précédente où dans ce cas on retire $\pi$ à Arctg Y/X. Pour la mesure $f_{m4}$, proche mais supérieure à $F_r/2$, Y est devenu négatif, la valeur de l'argument de Z change de signe. On réobtient alors la courbe en trait plein à partir de la courbe en pointillés en ajoutant $\pi$ à Arctg Y/X. En conséquence dans l'invention on calcule toujours $\theta$ tel que $\theta$ égale Arg ($\varepsilon$Z) selon la convention qui vient d'être indiquée pour passer de la courbe en pointillés à la courbe en trait plein.

Le choix de la valeur du seuil, ici $F_r/3$, à partir duquel on décide de moduler est imposé par le critère suivant: on cherche à obtenir la plage de variation la plus grande possible du signal mesuré pour que l'évolution de la mesure autour de cette valeur engendre le moins possible de permutations entre des situations où l'on module et des situations où l'on ne module pas. Il faut savoir pour comprendre ce critère

EP 0 161 956 B1

que ce seuil est un seuil donné en valeur absolue. Ceci signifie que l'on passera d'une situation à l'autre dès que la valeur obtenue, avec ou sans modulation selon le cas, dépassera négativement ou positivement cette valeur de seuil. Le décalage en fréquence apporté sur la mesure $f_m$ est proportionnel à $\pm\pi$, il est donc égal à $\pm F_r/2$.

Le choix d'un seuil à $F_r/4$ amène après décalage à transformer $f_m$ proche de $+F_r/4$ en $f_d$ proche de $-F_r/4$. Si le glissement Doppler à mesurer évolue au-dessus et en dessous de $F_r/4$ on sera constamment en train de changer d'une situation avec modulation à une situation sans modulation et vice versa. Il y a donc intérêt à éloigner ce seuil le plus possible de $F_r/4$. Si l'on choisit $F_r/2$ comme seuil, c'est un maximum puisque bien entendu Arg (Z) calculé par le circuit 10 ne franchit jamais cette valeur. Mais on se retrouve ici confronté à un autre problème. Dans ce cas en effet la plage de variation est maximum ce qui est favorable, mais l'intervention des filtres passe-bas 18 et 19 a pour effet de beaucoup perturber le signal S(t) démodulé autour de ces valeurs de glissement Doppler. En effet, ces filtres coupent justement à $F_r/2$ pour bien respecter les conditions d'un bon échantillonnage imposées par le théorème de Shannon. En définitive ceci reviendrait à passer d'une situation démodulée à une situation non modulée pour des valeurs de seuil qui ne sont pas atteintes d'une manière rigoureuse par le glissement Doppler à mesurer. Par ailleurs, il faut se souvenir que ces glissements Doppler ne sont pas des glissements Doppler monofréquence mais sont des glissements Doppler moyens calculés sur des spectres relativement étendus comme représentés en figure 2. Une valeur préférée de seuil est $F_r/3$. Une autre valeur préférée est 3 $F_r/8$. L'explication qui suit est faite pour $F_r/3$.

Si l'on appelle $\Delta\theta$ la correction qu'il faut appliquer à la courbe en trait plein qui représente Arg ($\varepsilon$Z), pour obtenir la courbe en tirets qui représente la valeur vraie de la vitesse à mesurer on peut calculer $\Delta\theta$ selon un organigramme représenté sur la figure 6. Le glissement Doppler vrai sera alors exprimé par:

$$f_v = \frac{F_r}{2\pi}\theta + F_r \cdot \Delta\theta$$

Au début du traitement, à $t$ inférieur à t1, c'est-à-dire lorsque l'on est sûr que le glissement Doppler mesuré est un glissement Doppler vrai et non un glissement Doppler ambigu d'un glissement Doppler vrai on fait $\varepsilon=1$ et $\Delta\theta=0$. Puis on calcule $\theta=$Arg($\varepsilon$Z) ce qui revient à calculer argument de Z puisque $\varepsilon$ vaut 1 (Figure 6). Ceci est encore équivalent à prendre pour bon le résultat $f_m$, délivré par l'analyseur 10 c'est-à-dire sans avoir à moduler. Puis on calcule la valeur vraie $f_v$ selon la formule précédente. On compare ensuite, en valeur absolue, la valeur de $\theta$ ainsi élaborée à une valeur correspondant au seuil. Si le seuil est $F_r/3$ la valeur absolue de $\theta$ est comparée à $2\pi/3$. Si valeur absolue de $\theta$ est inférieure à $2\pi/3$, on ne change ni $\varepsilon$ ni $\Delta\theta$. A la valeur suivante délivrée par l'analyseur 10 on recommence cette opération mais en prenant l'algorithme en aval des instructions qui ont imposé $\varepsilon$ à 1 et $\Delta\theta$ à 0.

En suivant le premier segment de la courbe en trait plein, on se rend compte qu'à un instant $t_1$, $f_m$ devient supérieur à $F_r/3$. La valeur de $f_v$ est néanmoins calculée comme précédemment. Cependant en comparant $\theta$ à $2\pi/3$ on est alors amené à se rendre compte que $\theta$ y est supérieur. On change alors le signe de $\varepsilon$ qui dans le cas présent devient égal à $-1$ et on effectue un test sur le signe de $\theta$. Ici $\theta$ est positif et on fait $\Delta\theta=\Delta\theta+1/2$. A la valeur suivant celle qui a été élaborée à $t_1$, on calcule $\theta=$Arg$\varepsilon$Z=Arg$-$Z qui correspond à la valeur décalée $f_d$. Ceci signifie que l'on est dans une situation avec modulation. On se trouve alors sur le deuxième segment de la courbe en trait plein de la figure 5. La correction $\Delta\theta$ n'est plus nulle, mais vaut alors 1/2 et on corrige bien de $+F_r/2$, la valeur dite décalée $f_d$ qui vaut $F_r \cdot \theta/2\pi$. A l'instant $t_2$, Arg($\varepsilon$Z) vaut zéro et rien ne se passe pour la correction $\Delta\theta$ ni pour $\varepsilon$ mais tout simplement Arg(Z) change de valeur selon la convention évoquée plus haut. Ceci est donc sans incidence sur le fonctionnement de l'algorithme de la figure 6 puisque maintenant on calcule Arg($\varepsilon$Z) qui lui passe par zéro.

A l'instant $t_3$ le nouveau $\theta$ calculé devient supérieur une deuxième fois à $2\pi/3$. Dans ces conditions on change à nouveau le signe de $\varepsilon$ qui devient maintenant $+1$. Comme $\theta$ est toujours supérieur à zéro (la courbe en trait plein à cet instant est supérieure à zéro) on fait $\Delta\theta=\Delta\theta+1/2$ soit $\Delta\theta=1$. Autrement dit d'une part on ne module plus, mais d'autre part le $\Delta\theta$ calculé correspond à avoir décalé deux fois dans le même sens: la correction tient compte des deux décalages successifs. A l'instant $t_4$ on décale même une troisième fois ce qui fait que $\Delta\theta$ devient égal à 3/2. Par contre à l'instant t5, $\theta$, qui est négatif, devient supérieur en valeur absolue à la valeur $2\pi/3$. On effectue les mêmes opérations qu'auparavant sur $\varepsilon$ mais ce qu'il y a d'intéressant à remarquer c'est que dans ce cas on fait $\Delta\theta=\Delta\theta-1/2$ puisque maintenant $\theta$ est négatif. En conséquence, la correction apportée, qui était auparavant de 3/2, ne vaut plus maintenant que 1. Ainsi de suite la correction évolue cumulativement par addition ou par soustraction le cas échéant.

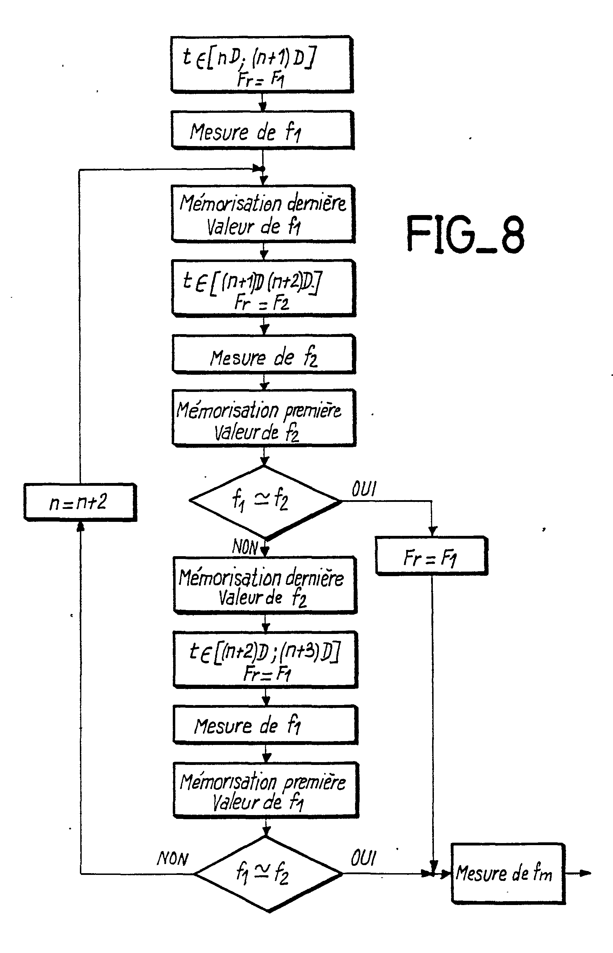

On a dit précédemment que l'on partait d'une situation où l'on était certain que la mesure de la vitesse n'était pas une mesure ambiguë mais était une mesure vraie. Or quand l'expérimentation commence il n'est pas certain justement que l'on se trouve dans cette situation. En conséquence, il peut s'avérer utile de faire précéder le procédé de levée d'ambiguïté de l'invention d'une phase d'initialisation où, en définitive, on acquiert la certitude que l'on est en train de mesurer des vitesses vraies et non des vitesses ambiguës de vitesses vraies. Cette phase d'initialisation va être décrite maintenant au moyen de la figure 8 qui représente son organigramme.

Le principe mis en oeuvre dans cette initialisation consiste à choisir des intervalles de temps successifs

8

pendant lesquels la fréquence de récurrence prend des valeurs différentes. Puis en comparant le résultat obtenu sur la mesure de la vitesse à la fin d'un intervalle avec le résultat obtenu au début de l'intervalle suivant on peut dire, si ces résultats sont sensiblement les mêmes, que la vitesse mesurée dans un cas comme dans l'autre, et qui n'a pas eu le temps de suffisamment changer de la fin d'un intervalle au début du suivant, est une vitesse vraie. En effet, il apparaît que si les mesures effectuées étaient des mesures ambiguës de vitesses vraies, du fait que les fréquences de récurrence sont différentes, elles devraient être différentes d'un cas à l'autre. Dans un exemple on retient deux fréquences, $F_1$ et $F_2$, qui imposent le rythme des impulsions alternativement dans un intervalle de temps et dans le suivant. Il convient de remarquer cependant que ce changement de fréquence de récurrence n'altère pas le fonctionnement de l'analyseur 10 décrit puisque celui-ci est piloté par un séquenceur 27 qui est justement synchronisé sur la période de récurrence elle-même.

Dans le domaine médical où l'on mesure la vitesse du sang, les différents intervalles d'analyse, avec l'une ou l'autre fréquence, seront liés au rythme cardiaque du patient. En considérant par exemple une durée minimale de pulsations cardiaques égale à 0,3 seconde (ce qui est équivaut à un rythme cardiaque de l'ordre de 200 pulsations par minute et qui semble de ce point de vue être une valeur limite) on est amené à la considération suivante: il faut pouvoir vérifier l'égalité des vitesses au cours d'un même cycle cardiaque et non pas d'un cycle cardiaque à un autre. En conséquence, des intervalles successifs de 0,1 seconde (10 Hz) permettent d'affirmer qu'il y a de grandes chances pour que, lors du passage d'un intervalle à un autre, la variation de vitesse du sang soit relativement faible. Avec une fréquence de récurrence valant par exemple alternativement 6 KHz et 8 KHz on a pu acquérir au cours de chaque intervalle de 0,1 seconde respectivement 600 ou 800 échantillons.

Comme il faut environ une centaine d'échantillons pour élaborer une mesure du glissement Doppler moyen, mais comme les mesures sont cependant données en temps réel par l'analyseur 10 au rythme de l'échantillonnage, il convient de comparer la dernière valeur acquise au cours d'un intervalle donné à la première valeur acquise dans le suivant pour laquelle la totalité des échantillons n'est intrinsèquement dépendante que de la nouvelle fréquence de récurrence retenue. En pratique, il faut donc attendre la réception du $P^{ème}$ échantillon dans le nouvel intervalle pour élaborer la "première" valeur de glissement Doppler moyen qui correspond à cette intervalle.

Il n'est pas nécessaire que les intervalles en question soient répartis périodiquement dans le temps. Les dates qui séparent les différentes intervalles peuvent être choisies aléatoirement. Il est même éventuellement possible de déterminer ces dates de changement de fréquence de récurrence dès que le glissement Doppler moyen $f_m$ atteint et dépasse un certain seuil. Cependant, l'organigramme de la figure 7 correspond à des intervalles de durée D tous égaux entre eux. Ainsi, au cours d'un premier intervalle lorsque la date de mesure $t$ est comprise entre $nD$ et $(n+1)D$, on impose que la fréquence de la récurrence $F_r$ soit égale à $F_1$. On mesure les glissements Doppler moyens pendant cette durée: leur valeur est $f_1$. A la fin de cette durée on mémorise la dernière valeur de $f_1$. Pendant la durée suivante, pour des dates comprises entre $(n+1)D$ et $(n+2)D$, on impose que la fréquence de récurrence soit égale à $F_2$. On mesure alors le glissement Doppler moyen obtenu selon cette nouvelle fréquence de récurrence, leur valeur est $f_2$. On mémorise la première valeur de $f_2$ qui est complètement découplée de la mesure de $f_1$ (c'est-à-dire P échantillon plus tard). Dès que $f_2$ est acquis on compare $f_2$ à $f_1$. Si ces deux valeurs sont égales on en déduit que toutes deux sont des valeurs vraies et on est donc dans une situation initialisée. Dans ces conditions on impose que la fréquence de récurrence prenne une des deux valeurs que l'on avait choisies pour cette initialisation: par exemple $F_1$. A ce moment on entre dans le processus de calcul décrit par l'algorithme de la figure 6 au cours duquel on lèvera les ambiguïtés qui pourront survenir sur la mesure de vitesse du mobile au fur et à mesure où elles se présenteront.

Par contre, si $f_1$ est différent de $f_2$ cela signifie que l'une au moins de ces mesures est ambiguë. Dans ces conditions on n'est pas encore capable de prendre de décision puisqu'on ne sait pas laquelle de ces deux mesures est une mesure vraie et si même l'une d'entre elles est une mesure vraie. En conséquence, on réitère l'ensemble des opérations d'initialisation effectuées jusqu'ici mais en mémorisant cette fois la dernière valeur mesurée de $f_2$. Au cours de l'intervalle suivant, après avoir changé la fréquence de récurrence de $F_2$ en $F_1$ on mesure et on mémorise la "première" valeur de $f_1$. Puis on compare à nouveau les dernières valeurs mémorisées de $f_1$ et de $f_2$. Si elles sont égales on peut affirmer que l'on est initialisé, si elles ne sont pas égales on recommence la totalité de l'opération jusqu'à ce que en définitive on obtienne l'initialisation.

On peut faire les remarques suivantes qui prévalent dans le cas de l'application médicale de l'invention. Quand on regarde un diagramme temporel des vitesses de circulation du sang on se rend compte que statistiquement ces vitesses de circulation sont plus souvent faibles que élevées. Les chances que l'on a de trouver des vitesses faibles, donc vraies, sont donc importantes. C'est la raison pour laquelle le processus d'initialisation qui vient d'être décrit fonctionne bien. Ceci ne serait pas nécessairement le cas d'un phénomène où les vitesses de déplacement seraient élevées et qui engendrerait statistiquement souvent des mesures ambiguës de la vitesses vraies. Dans ces dernières conditions la phase d'initialisation durerait plus longtemps mais finirait quand même par accrocher à un moment donné une valeur vraie. Le processus décrit dans la figure 7 permet d'accrocher la valeur vraie avec certitude, approximativement sur deux cycles cardiaques successifs, ce qui est somme toute satisfaisant.

Il faut reconnaître cependant que cette initialisation peut être faussée. En effet il peut arriver que $f_1$ soit

9

## EP 0 161 956 B1

égal à $f_2$ alors que chacune de ces mesures est une mesure ambiguë d'une vitesse vraie. Ceci signifie que la vitesse vraie correspondrait alors au plus petit commun multiple des deux fréquences de récurrence retenues. Dans l'exemple il s'agirait de glissement Doppler supérieur à 24 KHz. Une méthode simple pour repousser au plus haut possible ce seuil d'ambiguïté pour lequel l'initialisation est incorrecte consiste à choisir des fréquences $F_1$ et $F_2$ qui soient des nombres premiers entre eux par exemple 7 KHz et 8 KHz. Ceux-ci porterait l'ambiguïté en glissement Doppler à 56 KHz ce qui pour des fréquences acoustiques retenues dans cette description correspondrait à des vitesses de circulation du sang de l'ordre de 20 m par seconde et qui seraient véritablement exceptionnelles. Il faut cependant que chacune des fréquences $F_1$ et $F_2$ soit un sous multiple exact de la fréquence acoustique $f_0$.

La phase d'initialisation peut cependant être supprimée. En particulier, dans une application médicale, on remarque qu'au cours d'un cycle cardiaque la vitesse instantanée est faible pendant des durées relativement longues à la fin de ce cycle cardiaque. Or à vitesse faible le rapport signal à bruit de S(t) est plus mauvais à cause de la présence de filtres d'écho fixe. Ces filtres tendent à éliminer dans S(t) la contribution spectrale due par exemple aux parois des capilaires. En conséquence au cours d'un cycle cardiaque lorsque l'énergie du signal Doppler utile devient faible pendant relativement longtemps on est en présence de vitesse faible et donc non ambiguë. Pour se passer de l'initialisation on synchronise alors l'algorithme de correction d'ambiguïté sur une telle période.

**Revendications**

1. Procédé de levée d'ambiguïté de la mesure de la vitesse V d'un mobile (1) par effet Doppler, qui comprend les étapes suivantes:
   l'émission (6, 7, 8) en direction du mobile d'un signal acoustique de fréquence fo, modulé en impulsions, la fréquence de récurrence des impulsions étant Fr,
   la réception (50, 9) du signal acoustique S(t) rétrodiffusé par le mobile,
   la démodulation complexe (9) du signal S(t) par un signal à la fréquence d'émission fo pour obtenir un signal démodulé Z(t),
   l'extraction, par analyse spectrale (10) du signal démodulé Z(t), de la composante à la fréquence Doppler représentative de la vitesse du mobile,
   ledit procédé étant caractérisé en ce qu'il comprend, pour éliminer l'ambiguïté de mesure dûe à l'échantillonnage du signal démodulé Z(t) à la fréquence de récurrence Fr,
   a) la comparaison de la fréquence Doppler issue des moyens d'analyse spectrale (10) à une valeur de seuil donnée liée à l'apparition de l'ambiguïté, et en cas de dépassement du seuil, les étapes suivantes effectuées selon un processus récursif;
   b) la modulation (11) du signal démodulé Z(t) par un signal oscillant à une fréquence de détermination FD, de manière à obtenir une fréquence Doppler transposée fa d'une grandeur connue dans une bande de fréquence inférieure à la moitié de la fréquence de récurrence Fr;
   c) l'extraction, par analyse spectrale (10), de la fréquence Doppler transposée fa et la correction de ladite fréquence Doppler transposée par une grandeur fc correspondant à la fréquence de détermination pour calculer la fréquence Doppler vraie fv;
   d) la comparaison de la fréquence Doppler transposée à la valeur de seuil donnée et la poursuite du processus à partir de l'étape (b) précédente en cas de dépassement du seuil, et ceci jusqu'à l'obtention d'une fréquence Doppler vraie fv non ambiguë.

2. Procédé selon la revendication 1 caractérisé en ce que la fréquence de détermination est égale à la moitié de la fréquence de récurrence.

3. Procédé selon l'une quelconque des revendications 1 ou 2 caractérisé en ce que la modulation consiste à multiplier le signal démodulé par le signal à la fréquence de détermination.

4. Procédé selon l'une quelconque des revendications 1 à 3 caractérisé en ce que l'analyse spectrale (10) comporte l'élaboration (22) de la fonction d'autocorrélation (X, Y) du signal modulé (Z(t)) et le calcul (23) de la dérivée d'ordre un (Arg(Z)) de cette fonction.

5. Procédé selon l'une quelconque des revendications 1 à 3 caractérisé en ce que l'analyse spectrale (10) comporte l'élaboration (22) de la fonction d'autocorrélation (X, Y) du signal démodulé (Z(t)) et le calcul (23) de la dérivée d'ordre un (Arg(Z)) de cette fonction et en ce que la modulation du signal démodulé est remplacée par une modification (Arg($\varepsilon$Z)) apportée à ce résultat sous la forme d'une constante ($\pm\pi$) de transposition qui tient lieu de modulation.

6. Procédé selon l'une quelconque des revendications 1 à 5 caractérisé en ce que la correction ($f_c$) est apportée à la fréquence transposée sous la forme de constantes ($\Delta\theta$) additives ou soustractives, calculées par avance, et dont les valeurs dépendent du rapport liant la fréquence de détermination à la fréquence de récurrence, ces corrections étant additives quand la fréquence Doppler transposée ($\theta$) est positive et étant négatives quand la fréquence Doppler transposée ($\theta$) est négative.

7. Procédé selon l'une quelconque des revendications 1 à 6 caractérisé en ce qu'il est précédé d'une étape d'initialisation (Fig. 8) dans laquelle la fréquence de récurrence prend des valeurs différentes ($F_1$, $F_2$) au cours d'intervalles de temps successifs les uns des autres et en ce que l'on compare entre elles la dernière mesure de vitesse ($f_1$) élaborée à la fin d'un intervalle à la première mesure de vitesse ($f_2$) élaborée

10

# EP 0 161 956 B1

au début de l'intervalle suivant pour déterminer qu'aucune de ces mesures n'est ambiguë quand elles sont sensiblement égales entre elles.

8. Procédé selon l'une quelconque des revendications 1 à 7 caractérisé en ce que le seuil donné correspond autiers de la fréquence de récurrence $F_r$.

9. Procédé selon l'une des revendications 1 à 7 caractérisé en ce que le seuil donné correspond aux trois huitièmes de la fréquence de récurrence.

10. Procédé selon la revendication 8 caractérisé en ce que la correction est élaborée cumulativement en fonction du sens des évolutions de la fréquence transposée autour du seuil donné.

11. Procédé selon la revendication 7 caractérisé en ce que les intervalles de temps sont de durée quelconque.

12. Procédé selon la revendication 7 caractérisé en ce que les intervalles de temps sont égaux entre eux (D).

13. Procédé selon la revendication 7 caractérisé en ce que les intervalles de temps sont déterminés par des dates qui marquent le passage des mesures de fréquence transposée à des valeurs prédéterminées.

## Patentansprüche

1. Verfahren zum Auflösen von Mehrdeutigkeiten bei der Messung der Geschwindigkeit V eines bewegten Objektes (1) durch den Dopplereffekt, das die folgenden Schritte umfaßt:

das in Richtung des beweglichen Objektes erfolgende Aussenden (6, 7, 8) eines in Form von Impulsen modulierten akustischen Signals der Frequenz fo, wobei die Folgefrequenz der Impulse Fr ist,

das Empfangen (50, 9) des durch das bewegliche Objekt rückgestreuten akustischen Signals S(t),

die komplexe Demodulation (9) des Signals S(t) durch ein Signal mit der Sendefrequenz fo, um ein demoduliertes Signal Z(t) zu erhalten,

die Extraktion des demodulierten Signals Z(t) der Komponente mit der Dopplerfrequenz, die für die Geschwindigkeit des beweglichen Objektes repräsentativ ist, durch Spektralanalyse (10);

wobei das gennante Verfahren dadurch gekennzeichnet ist, daß es zur Auflösung von Mehrdeutigkeiten bei der Messung, die durch die Abtastung des demodulierten Signals Z(t) mit der Folgefrequenz Fr verursacht sind, umfaßt:

a) den Vergleich zwischen der durch die Mittel (10) zur Spektralanalyse gewonnenen Dopplerfrequenz und einem vorgegebenen Schwellenwert, der mit dem Auftreten der Mehrdeutigkeiten verknüpft ist, und im Falle des Überschreitens der Schwelle die folgenden Schritte umfaßt, welche gemäß eines rekursiven Vorganges erfolgen;

b) die Modulation (11) des demodulierten Signals Z(t) durch ein mit einer Bestimmungsfrequenz FD schwingendes Signal, so daß eine um einen bekannten Wert umgesetzte Dopplerfrequenz fa in einem Frequenzbereich erhalten wird, das kleiner ist als die Hälfte der Folgefrequenz Fr;

c) die durch Spektralanalyse (10) erfolgende Extraktion der umgesetzten Dopplerfrequenz fa sowie die Korrektur der genannten umgesetzten Dopplerfrequenz durch eine Größe fc, die der Bestimmungsfrequenz entspricht, um die echte Dopplerfrequenz fv zu berechnen;

d) den Vergleich zwischen der umgesetzten Dopplerfrequenz und dem vorgegebenen Schwellenwert sowie das Fortsetzen des Vorgangs ab dem vorausgehenden Schritt (b), falls die Schwelle überschritten wird, und dies, bis eine eindeutige echte Dopplerfrequenz fv erhalten wird.

2. Verfahren nach Anspruch 1, dadurch gekennzeichnet, daß die Bestimmungsfrequenz gleich der Hälfte der Folgefrequenz ist.

3. Verfahren nach einem der Ansprüche 1 und 2, dadurch gekennzeichnet, daß die Modulation darin besteht, das demodulierte Signal mit dem Signal der Bestimmungsfrequenz zu multiplizieren.

4. Verfahren nach einem der Ansprüche 1 bis 3, dadurch gekennzeichnet, daß die Spektralanalyse (10) das Erzeugen (22) der Autokorrelationsfunktion (X, Y) des modulierten Signals (Z(t)) sowie die Berechnung (23) der Ableitung erster Ordnung (Arg(Z)) dieser Funktion umfaßt.

5. Verfahren nach einem der Ansprüche 1 bis 3, dadurch gekennzeichnet, daß die Spektralanalyse (10) das Erzeugen (22) der Autokorrelationsfunktion (X, Y) des demodulierten Signals (Z(t)) sowie die Berechnung (23) der Ableitung erster Ordnung (Arg(Z)) dieser Funktion umfaßt, und daß die Modulation des demodulierten Signals durch eine Veränderung (Arg($\varepsilon$Z)) ersetzt wird, die an diesem Ergebnis in Form einer Transpositionskonstante ($\pm\pi$) vorgenommen wird, die an die Stelle einer Modulation tritt.

6. Verfahren nach einem der Ansprüche 1 bis 5, dadurch gekennzeichnet, daß die Korrektur ($f_c$) an der umgesetzte Frequenz in Form von Konstanten ($\Delta\theta$) vorgenommen wird, die additiv oder subtraktiv sind und im voraus berechnet werden und wovon die Werte von dem Verhältnis abhängen, das die Bestimmungsfrequenz mit der Folgefrequenz verknüpft, wobei die Korrekturen additiv sind, wenn die umgesetzte Dopplerfrequenz ($\theta$) positiv ist und negativ sind, wenn die umgesetzte Dopplerfrequenz ($\theta$) negativ ist.

7. Verfahren nach einem der Ansprüche 1 bis 6, dadurch gekennzeichnet, daß ihm ein Initialisierungsschritt (Fig. 8) vorausgeht, in welchem die Folgefrequenz verschiedene Werte ($F_1$, $F_2$) während aufeinanderfolgenden Zeitintervallen annimmt, und daß ein Vergleich vorgenommen wird zwischen der letzten Geschwindigkeitsmessung ($f_1$), die am Ende eines Intervalls erzeugt wird, und der

11

ersten Geschwindigkeitsmessung (f$_2$), die am Anfang des folgenden Intervalls erzeugt wird, um festzustellen, daß keine dieser Messungen mehrdeutig ist, wenn sie im wesentlichen einander gleich sind.

8. Verfahren nach einem der Ansprüche 1 bis 7, dadurch gekennzeichnet, daß die vorgegebene Schwelle dem Drittel der Folgefrequenz Fr entspricht.

9. Verfahren nach einem der Ansprüche 1 bis 7, dadurch gekennzeichnet, daß die vorgegebene Schwelle den Dreiachteln der Folgefrequenz entspricht.

10. Verfahren nach Anspruch 8, dadurch gekennzeichnet, daß die Korrektur in Abhängigkeit von der Entwicklungsrichtung der umgesetzten Frequenz um die vorgegebene Schwelle kumulativ verarbeitet wird.

11. Verfahren nach Anspruch 7, dadurch gekennzeichnet, daß die Zeitintervalle eine beliebige Dauer haben.

12. Verfahren nach Anspruch 7, dadurch gekennzeichnet, daß die Zeitintervalle untereinander gleich sind (D).

13. Verfahren nach Anspruch 7, dadurch gekennzeichnet, daß die Zeitintervalle durch Zeitdaten bestimmt werden, die den Durchgang der Messungen der umgesetzten Frequenz durch vorbestimmte Werte markieren.

## Claims

1. A method of overcoming ambiguity in the measurement of velocity V of a moving object (1) by the doppler effect, which comprises the following steps:

the transmission (6, 7 and 8) towards the moving object of an acoustic signal of a frequency fo, with pulse modulation, the recurrent frequency of the pulses being Fr,

receiving (50 and 9) the acoustic signal S(t) emitted backwards by the moving object,

complex demodulation (9) of the signal S(t) using a signal at the transmission frequency fo in order to obtain a demodulated signal Z(t), and

extracting, using spectral analysis (10), the demodulated signal Z(t), of the component at the doppler frequency representative of the velocity of the moving object,

the said method being characterized in that it comprises, in order to eliminate the ambiguity of the measurement due to the sampling of the demodulated signal Z(t) at the recurrent frequency Fr,

(a) comparing the doppler frequency provided by the spectral analysis means (10) with a given threshold value related to the appearance of the ambiguity, and, if said threshold is exceeded, the following steps performed using a recursive process;

(b) modulation (11) of the demodulated signal Z(t) using a signal oscillating at a determination frequency FD, in such a manner as to obtain a transposed doppler frequency fa of a known magnitude in a frequency band lower than half the recurrent frequency Fr;

(c) extracting, by spectral analysis (10), the transposed doppler frequency fa and correcting the said transposed doppler frequency using a magnitude fc corresponding to the frequency of determination in order to calculate the true doppler frqeuency fv; and

(d) comparing the transposed doppler frequency with the given threshold value and performance of the process as from the step (b) if such threshold has been exceeded, this being performed until a non-ambiguous doppler frequency fv has been obtained.

2. The method as claimed in claim 1, characterized in that the determination frequency is equal to half the recurrent frequency.

3. The method as claimed in claim 1 or claim 2, characterized in that the modulation involves multiplying the demodulated signal by the signal oscillating at the determination frequency.

4. The method as claimed in any one of the claims 1 through 3, characterized in that the spectral analysis (10) comprises the elaboration (22) of the function of autocorrelation (X and Y) of the modulated signal (Z(t)) and the calculation (23) of the derivation (Arg(Z)) with an order of unity of this function.

5. The method as claimed in any one of the claims 1 through 3, characterized in that the spectral analysis (10) includes the elaboration (22) of the function (X and Y) of autocorrelation of the demodulated signal (Z(t)) and the calculation (23) of the derivation (Arg(Z)) with an order of unity of this function and in that the modulation of the demodulated signal is replaced by a modification (Arg($\varepsilon$Z)) added to this result in the form of a transposition constant ($\pm\pi$) which takes the place of modulation.

6. The method as claimed in any one of the claims 1 through 5, characterized in that the correction (f$_c$) is carried out on the transposed velocity in the form of additive or subtractive constants ($\Delta$ and $\theta$), calculated in advance, whose values depend on the ratio between the determination frequency and the recurrent frequency, such corrections being additive when the transposed doppler frequency ($\theta$) is positive and negative when the transposed doppler frequency ($\theta$) is negative.

7. The method as claimed in any one of the claims 1 through 6, characterized in that it is preceded by an initialization step (figure 8) in which the recurrent frequency assumes different values (F$_1$ and F$_2$) in the course of mutual successive intervals of time and in that the last measurement (f$_1$) of velocity elaborated at the end of an interval is compared with the first measurement of velocity (f$_2$) elaborated at the beginning of the following interval in order to ascertain that neither of these measurements is ambiguous when they are substantially equal to each other.

8. The method as claimed in any one of the claims 1 through 7, characterized in that the given threshold corresponds to a third of the recurrent frequency (Fr).

9. The method as claimed in any one of the claims 1 through 7, characterized in that the given threshold corresponds to three eighths of the recurrent frequency.

10. The method as claimed in claim 8, characterized in that the correction is elaborated cumulatively as a function of the direction of the evolution of the tranposed frequency about the given threshold.

11. The method as claimed in claim 7, characterized in that the intervals of time are of random duration.

12. The method as claimed in claim 7, characterized in that time intervals are equal to each other (D).

13. The method as claimed in claim 7, characterized in that the time intervals are determined by the data which signify the coincidence of the measures of the transposed frequency with predetermined values.

# FIG_1

FIG_2

$z(f)$  $Fr$

$-Fr$  $-Fr/2$  $f_M$  $0$  $Fr/2$  $f_N$  $Fr$  $f$

FIG_3-a

$f_v$

$Fr/2$

$t$

FIG_3-b

$f_m$

$F_R/2$

$f_{mi}$

$-Fr/2$

$t$

EP 0 161 956 B1

**EP 0 161 956 B1**

# FIG_4

FIG_5

# FIG_6

# FIG_7

FIG_8